(19) Europäisches Patentamt
European Patent Office
Office européen des brevets

(11) Veröffentlichungsnummer: **0 008 113**
**B1**

(12) # EUROPÄISCHE PATENTSCHRIFT

(45) Veröffentlichungstag der Patentschrift: **19.01.83**

(51) Int. Cl.³: **C 07 D 303/44,** C 07 D 301/14

(21) Anmeldenummer: **79102858.2**

(22) Anmeldetag **08.08.79**

(54) **Verfahren zur Herstellung von 7-Oxabicyclo (4.1.0.)heptan-3,4-dicarbonsäurediglycidylester.**

(30) Priorität: **16.08.78 DE 2835885**

(43) Veröffentlichungstag der Anmeldung: **20.02.80 Patentblatt 80/4**

(45) Bekanntmachung des Hinweises auf die Patenterteilung: **19.01.83 Patentblatt 83/3**

(84) Benannte Vertragsstaaten: **CH DE FR GB IT NL**

(56) Entgegenhaltungen:
**DE-A-1 942 557**
**DE-A-2 602 776**
**DE-B-1 082 263**
**DE-B-1 083 797**
**FR-A-1 269 628**

(73) Patentinhaber: **BAYER AG, Zentralbereich Patente, Marken und Lizenzen, D-5090 Leverkusen 1, Bayerwerk (DE)**

(72) Erfinder: **Rauleder, Gebhard, Dr., Mozartstrasse 20, D-5657 Haan (DE)**
Erfinder: **Waldmann, Helmut, Dr., Carl-Rumpff-Strasse 59, D-5090 Leverkusen 1 (DE)**
Erfinder: **Bottenbruch, Ludwig, Dr., Woehlerstrasse 5, D-4150 Krefeld 1 (DE)**
Erfinder: **Traenckner, Hans-Joachim, Dr., Wedelstrasse 46, D-4150 Krefeld 1 (DE)**
Erfinder: **Seifert, Hermann, Dr., Ruwergasse 4, D-5000 Koeln 80 (DE)**
Erfinder: **Swodenk, Wolfgang, Dr., Auf dem Broich 5, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen)

EP 0 008 113 B1

Verfahren zur Herstellung von
7-Oxabicyclo(4.1.0)heptan-3,4-dicarbonsäurediglycidylester

Die vorliegende Erfindung betrifft ein Verfahren zur Herstellung von 7-Oxabicyclo(4.1.0)heptan-3,4-dicarbonsäurediglycidylester aus Tetrahydrophthalsäurediglycidylester.

7-Oxabicyclo(4.1.0)heptan-3,4-dicarbonsäurediglycidylester kann als Ausgangsprodukt zur Herstellung von Epoxyharzen, von Polyäthern und Polyurethanen, zur Herstellung von Lacken und Kunststoffen, als Weichmacher, als Stabilisator und als organisches Zwischenprodukt Verwendung finden (s. z. B. DE-B-1 082 263, US-A-2 870 179 und Proc. Anniv. Conf. SPI (Soc. Plast. Ind.) Reinf. Plast. Comp. Div. 25th, 1970, B-B, S. 1—8). Ein technischer Einsatz dieses Produktes ist in größerem Maße bisher nicht erfolgt, da kein befriedigendes technisches Verfahren zu seiner Herstellung zur Verfügung gestanden hat.

Eine Möglichkeit, Olefine in Epoxide umzuwandeln, besteht in der Anwendung der »Prileschajew-Reaktion« (vgl. N. Prileschajew, Ber. dtsch. chem. Ges. 42, 4811, [1909]).

Bekanntlich lassen sich niedere aliphatische Percarbonsäuren in einer Gleichgewichtsreaktion aus Carbonsäure und Wasserstoffperoxid gemäß Gleichung (1) herstellen. (D. Swern, in »Organic Peroxides«, Vol. 1, S. 61, Wiley Intersciense 1971)

$$RCOOH + H_2O_2 \rightleftarrows RCOOOH + H_2O \qquad (1)$$

Mit Ausnahme von stärkeren Carbonsäuren wie Ameisensäure und Trifluoressigsäure benötigt man zur schnellen Einstellung des Gleichgewichtes starke Säuren, wie Schwefelsäure, p-Toluolsulfonsäure und andere, als Katalysator (S. N. Lewis in R. L. Augustin »Oxidation«, Vol. I, S. 216 [»C. Peracids«], Marcel Dekker, New York 1966). Die Umsetzung von Olefinen mit z. B. nach dieser Methode hergestellter Peressigsäure führte jedoch nicht zu Oxiranen, sondern zu α-Glykolen und Hydroxyacetaten (J. Böseken, W. C. Smit und Gaster, Proc. Acad. Sci. Amsterdam, 32 377—383 [1929]). Die im Reaktionsgemisch vorhandene Mineralsäure katalysiert die Aufspaltung des primär gebildeten Oxirans (D. Swern »Organic Peroxides«, Wiley Intersciense 1971, Vol. 2, S. 436), was besonders bei Substanzen wie Tetrahydrophthalsäurediglycidylester, die bereits Epoxidgruppen im Molekül enthalten, zu Oxiranverlusten führen kann.

Tetrahydrophthalsäurediglycidylester läßt sich daher nicht ohne weiteres epoxidieren, z. B. wird in der US-A-2 794 030 die Epoxidation der verschiedensten Alkylester der Tetrahydrophthalsäure mit Peressigsäure beschrieben. Die Umsetzung von Tetrahydrophthalsäurediglycidylester wird jedoch nicht erwähnt.

Aus der DE-B-1 083 797 ist ein Verfahren zur Herstellung von Epoxiden durch Umsetzung von Monoolefinen mit 7 bis 12 C-Atomen mit Peressigsäure bekannt, bei dem man das Reaktionsgemisch in Gegenwart eines Überschusses an Olefin destilliert, der ausreicht, um die entstandene Essigsäure als azeotropes Gemisch zu entfernen. Diese Destillation wird bei Temperaturen unter 100°C durchgeführt, und man kann in Gegenwart eines über dem Siedepunkt des Epoxids siedenden Treibmittels arbeiten. Das erfindungsgemäße Verfahren unterscheidet sich von diesem Verfahren wesentlich. So entstehen erfindungsgemäß höher als Essigsäure siedende Carbonsäuren, von denen nicht bekannt ist, ob sie mit Tetrahydrophthalsäurediglycidylester Azeotrope bilden können. Solche Azeotrope hätten in jedem Fall aber wesentlich höhere Siedepunkte als die gemäß der DE-B-1 083 797 auftretenden. Der erfindungsgemäß zuzufügende Zusatzstoff soll zwischen der entstehenden Carbonsäure und dem nächst höher siedenden Bestandteil sieden, nicht oberhalb des gebildeten Epoxids. Erfindungsgemäß kann die Destillation nicht auf Temperaturen unter 100°C begrenzt werden, in der DE-B-1 083 797 wird dagegen ausgesagt, daß bereits über 50°C die gebildete Carbonsäure Epoxide aufspalten kann, und erfindungsgemäß liegen Tris-Epoxide vor, die erhöhte Angriffsmöglichkeiten haben im Vergleich zu den Monoepoxiden, die gemäß der DE-B-1 083 797 erhalten werden. Schließlich geht aus dieser Druckschrift nicht hervor, daß das dort beschriebene Verfahren zur Epoxidation von bereits Epoxygruppen enthaltenden Verbindungen geeignet ist. Aus allen diesen Gründen kann das Verfahren der DE-B-1 083 797 das erfindungsgemäße Verfahren nicht nahelegen.

Es wurde nun ein Verfahren zur Herstellung von 7-Oxabicyclo(4.1.0)heptan-3,4-dicarbonsäurediglycidylester gefunden, das dadurch gekennzeichnet ist, daß man Tetrahydrophthalsäurediglycidylester mit einer 3 bis 4 Kohlenstoffatome enthaltenden Percarbonsäure in Gegenwart von organischen Lösungsmitteln bei einer Temperatur von 0 bis 100°C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem Lösungsmittel abdestilliert.

Nach dem erfindungsgemäßen Verfahren einsetzbarer Tetrahydrophthalsäurediglycidylester kann in an sich bekannter Weise erhalten werden, beispielsweise durch Umsetzung von Tetrahydrophthalsäure mit Epihalogenhydrin (vgl. DE-B-1 211 177).

Als organische Lösungsmittel können im erfindungsgemäßen Verfahren die verschiedensten unsubstituierten und substituierten Kohlenwasserstoffe verwendet werden, die unter Reaktionsbedingungen flüssig sind und unerwünschte Nebenreaktionen nicht oder nur in ganz untergeordnetem Maße eingehen. Als Kohlenwasserstoffe können z. B. verwendet werden aliphatische und cycloaliphatische Kohlenwasserstoffe wie Hexan, Heptan, Octan, 2-Äthyl-hexan, Decan, Dodecan, Cyclohexan, Methylcyclopentan und Petroläther, weiterhin aromatische Kohlenwasserstoffe wie Benzol, Nitrobenzol, Toluol, Äthylbenzol, Cumol, Diisopropylbenzol, Xylol und Chlorbenzol, weiterhin sauerstoffhaltige Kohlenwasserstoffe wie Diäthyläther, Diisopropyläther, Dibutyläther, Tetrahydrofuran, Dioxan, Essigsäureäthylester, Essigsäuremethylester, Essigsäurepropylester, Essigsäurebutylester, Propionsäuremethylester, Propionsäureäthylester, Propionsäurepropylester, Buttersäuremethylester, Buttersäureäthylester, Buttersäurepropylester, Buttersäurebutylester, Benzoesäuremethylester und Benzoesäureäthylester, weiterhin chlorierte Kohlenwasserstoffe wie Methylenchlorid, Chloroform, Tetrachlorkohlenstoff, 1-Chloräthan, 1,2-Dichloräthan, 1,1-Dichloräthan, 1,1,2,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol, von den aliphatischen und cycloaliphatischen Kohlenwasserstoffen 2-Äthyl-hexan, Cyclohexan und Methylcyclopentan und von den sauerstoffhaltigen Kohlenwasserstoffen Tetrahydrofuran, Propionsäureäthylester und Benzoesäureäthylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Propionsäureäthylester.

Verwendet werden können auch Lösungsmittelgemische der verschiedenen oben angegebenen organischen Lösungsmittel.

Erfindungsgemäß verwendbare Percarbonsäuren sind Perpropionsäure, Perbuttersäure und Perisobuttersäure. Bevorzugt verwendet werden Perpropionsäure und Perisobuttersäure.

Besonders bevorzugt ist Perpropionsäure. Diese Percarbonsäuren, gelöst in einem der genannten organischen Lösungsmittel, können z. B. nach dem in der DE-A-2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Percarbonsäure mit einem organischen Lösungsmittel aus dem Reaktionsgemisch extrahiert wird. Gegebenenfalls kann die so erhaltene Percarbonsäurelösung in dem organischen Lösungsmittel noch weiter gereinigt werden, insbesondere um den Gehalt an Wasser, Wasserstoffperoxid und Schwefelsäure zu erniedrigen.

Im allgemeinen verwendet man die Percarbonsäuren in Form einer Lösung in einem organischen Lösungsmittel. Derartige Percarbonsäurelösungen können beispielsweise 10 bis 30 Gew.-% der jeweiligen Percarbonsäure, bezogen auf die Lösung, enthalten. Tetrahydrophthalsäurediglycidylester kann als solcher oder ebenfalls gelöst in einem oder mehreren der vorstehend genannten Lösungsmittel eingesetzt werden, wobei beliebig konzentrierte Lösungen zum Einsatz gelangen können, Vorzugsweise wird Tetrahydrophthalsäurediglycidylester als solcher eingesetzt und organische Lösungsmittel nur in Form der Percarbonsäurelösung zugegeben.

Das Molverhältnis von eingesetzter Percarbonsäure zu eingesetztem Tetrahydrophthalsäurediglycidylester kann in weiten Grenzen schwanken. Beispielsweise kann dieses Molverhältnis 0,1 : 1 bis 5 : 1 betragen. Bevorzugt wird ein Molverhältnis von 0,3 : 1 bis 3 : 1 angewendet. Ganz besonders vorteilhaft ist es, ein Molverhältnis von 0,5 bis 1,5 Mol Persäure je Mol Tetrahydrophthalsäurediglycidylester anzuwenden.

Der Wassergehalt der verwendeten Percarbonsäure soll im allgemeinen möglichst niedrig sein. Wassermengen bis 2 Gew.-% in der Percarbonsäurelösung sind im allgemeinen nicht störend. Geeignet ist beispielsweise eine Percarbonsäurelösung mit einem Wassergehalt von bis zu 1 Gew.-%. Vorzugsweise verwendet man eine Percarbonsäurelösung, die weniger als 0,3 Gew.-% Wasser enthält. Besonders bevorzugt ist ein Wassergehalt von weniger als 0,1 Gew.-%.

Der Wasserstoffperoxidgehalt der verwendeten Percarbonsäure in einem der oben genannten organischen Lösungsmittel soll im allgemeinen möglichst niedrig sein. Er kann beispielsweise bis zu 1 Gew.-%, bezogen auf die Percarbonsäurelösung, betragen. Vorteilhaft arbeitet man bei einem Gehalt von weniger als 0,5 Gew.-%. Besonders vorteilhaft ist es, die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäure soll möglichst niedrig sein. Vorteilhaft ist es, die Reaktion mit einer Percarbonsäurelösung durchzuführen, die einen Mineralsäuregehalt unterhalb 0,005 Gew.-% besitzt. Besonders vorteilhaft ist ein Mineralsäuregehalt von weniger als 0,001

Gew.-%.

Das erfindungsgemäße Verfahren wird in dem Temperaturbereich von 0—100°C durchgeführt. Bevorzugt arbeitet man bei 10—80°C, besonders bevorzugt bei 20—50°C. In Sonderfällen können die angegebenen Temperaturen auch unter- oder überschritten werden.

Neben der Arbeitsweise unter isothermen Bedingungen, d. h. Einhaltung einer einheitlichen Temperatur im gesamten Reaktionsgemisch, kann man das erfindungsgemäße Verfahren auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das erfindungsgemäße Verfahren kann bei den verschiedensten Drucken durchgeführt werden. Im allgemeinen arbeitet man bei Normaldruck. Das Verfahren kann jedoch auch bei Unter- oder Überdruck durchgeführt werden.

Die Durchführung des erfindungsgemäßen Verfahrens kann diskontinuierlich oder kontinuierlich in den für Umsetzungen dieser Art üblichen Vorrichtungen wie Rührwerkskessel, Siedereaktoren, Röhrenreaktoren, Schlaufenreaktoren oder Schleifenreaktoren erfolgen.

Als Werkstoffe für die Reaktionsapparate zur Durchführung des erfindungsgemäßen Verfahrens können beispielsweise Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die Schwermetallionen durch Komplexbildung inaktivieren können. Bekannte Substanzen solcher Art sind beispielsweise Gluconsäure, Äthylendiamintetraessigsäure, Natriumsilicat, Natriumpyrophosphat, Natriumhexametaphosphat, Dinatriumdimethylpyrophosphat oder $Na_2$(2-Äthyl-hexyl)$_5$($P_3O_{10}$)$_2$ (vgl. DE-B-1 056 596, Spalte 4, Zeile 60 ff.).

Die Reaktionswärme kann durch innen- oder außenliegende Kühler abgeführt werden. Zur Ableitung der Reaktionswärme kann die Umsetzung auch unter Rückfluß (z. B. in Siedereaktoren) durchgeführt werden.

Der Tetrahydrophthalsäurediglycidylester und die Percarbonsäure können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise der Tetrahydrophthalsäurediglycidylester vorgelegt und dann die Percarbonsäurelösung zugegeben. Die Reaktionstemperatur kann dabei vor oder nach der Zugabe der Percarbonsäure eingestellt werden. Man kann auch die beiden Komponenten bei Raumtemperatur gleichzeitig in das Reaktionsgefäß eingeben und dann die Reaktionstemperatur einstellen. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinander geschaltet sein können, kann es vorteilhaft sein, den Tetrahydrophthalsäurediglycidylester nur in den ersten Reaktor einzubringen. Man kann die Esterzugabe jedoch auch auf mehrere Reaktoren verteilen.

Es kann von Vorteil sein, den Tetrahyrophthalsäurediglycidylester durch chargenweise Zugabe der Percarbonsäure zu epoxidieren. Dabei kann es von Vorteil sein, nach Zugabe einzelner Chargen die aus der Percarbonsäure entstandene Carbonsäure aus dem Reaktionsgemisch zu entfernen, z. B. durch Extraktion mit Wasser oder durch Destillation.

Die nach Durchführung des erfindungsgemäßen Verfahrens erhaltenen Reaktionsgemische enthalten im allgemeinen das verwendete organische Lösungsmittel, die aus der Percarbonsäure entstehende Carbonsäure und 7-Oxabicyclo(4.1.0)heptan-3,4-dicarbonsäurediglycidylester, wobei gegebenenfalls unumgesetzter Tetrahydrophthalsäurediglycidylester sowie gegebenenfalls geringe Mengen von hochsiedenden Nebenprodukten im Reaktionsgemisch vorhanden sein können.

Die Aufarbeitung des erhaltenen Reaktionsgemisches erfolgt durch Destillation. Dabei kann man so verfahren, daß man die einzelnen Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung des Reaktionsgemisches ermöglichen, durchzuführen. Erfindungsgemäß wird dem Reaktionsgemisch ein geeigneter Zusatzstoff zugefügt, der zwischen der Carbonsäure und dem nächst höher siedenden Bestandteil des Reaktionsgemisches siedet. Dadurch erreicht man eine vollständige Abtrennung der Carbonsäure bei relativ niedrigen Sumpftemperaturen, was hinsichtlich der Minimierung der Bildung von Nebenprodukten aus der Carbonsäure und den epoxidierten Bestandteilen des Reaktionsgemisches von Vorteil ist. Erfindungsgemäß wird dann die Carbonsäure zusammen mit dem Lösungsmittel abdestilliert.

Es kann vorteilhaft sein, dem Reaktionsgemisch vor oder während der Aufarbeitung, insbesondere bei destillativer Aufarbeitung, Stabilisatoren zuzusetzen, welche die Bildung von Hochsiedern und Polymerisaten verhindern.

Das erfindungsgemäße Verfahren gestattet es erstmals, nach der sogenannten Prileschajew-Reaktion in technischem Maßstab 7-Oxabicyclo(4.1.0)heptan-3,4-dicarbonsäurediglycidylester aus Tetrahydrophthalsäurediglycidylester herzustellen.

Das folgende Beispiel erläutert die Erfindung. Sämtliche Prozentangaben sind, soweit nichts

anderes gesagt wird, Gewichtsprozente.

Beispiel

In einem 5-l-Dreihals-Doppelwand-Glaskolben mit Magnetrührer, Innenthermometer, Tropftrichter und Rückflußkühler wurden 1410 g Diglycidyltetrahydrophthalat (5 Mol) vorgelegt und auf 40°C thermostatisiert. Danach tropfte man 2700 g einer 20%igen Lösung von Perpropionsäure in Benzol (6 Mol), mit einem Wassergehalt von unter 0,1%, einem Wasserstoffperoxidgehalt von unter 0,2% und einem Mineralsäuregehalt von unter 0,001%, so schnell zu, daß die Temperatur bei 40°C gehalten werden konnte. Nach Zutropfende wurde weiter bei dieser Temperatur gerührt. In zeitlichen Abständen wurden dem Reaktionsgemisch Proben entnommen und der zum jeweiligen Zeitpunkt noch vorhandene Gehalt an Perpropionsäure durch Titration, die Bildung des Epoxides bzw. die Abnahme des Olefins gaschromatographisch durch Zuwiegen eines internen Standards bestimmt.

Nach einer Reaktionszeit von 3,5 Stunden betrug der Umsatz von Diglycidyltetrahydrophthalat 98%. 7-Oxabicyclo(4.1.0)heptan-3,4-dicarbonsäurediglycidylester wurde mit einer Selektivität von 91% bezogen auf umgesetzten Tetrahydrophthalsäurediglycidylester gebildet.

Zur Aufarbeitung wurden dem Reaktionsgemisch 2000 ml Benzoesäureäthylester zugefügt. Anschließend wurde das Gemisch in einer Füllkörperkolonne mit Fallstromverdampfer fraktioniert. Bei einem Vakuum von 200 mbar wurden Benzol und Propionsäure als gemeinsames Kopfprodukt erhalten; es wurde in einer weiteren Kolonne in die Komponenten aufgetrennt.

Das Sumpfprodukt wurde einer erneuten Destillation unterworfen. Bei einem Vakuum von 0,2 Torr wurde 7-Oxabicyclo(4.1.0)heptan-3,4-dicarbonsäurediglycidylester bei einer Kopftemperatur von 178—180°C erhalten. Die Epoxidtitration ergab einen Epoxidsauerstoffgehalt von 16,0% (theor. 16,1%).

**Patentansprüche**

1. Verfahren zur Herstellung von 7-Oxabicyclo(4.1.0)heptan-3,4-dicarbonsäurediglycidylester, dadurch gekennzeichnet, daß man Tetrahydrophthalsäurediglycidylester mit einer 3 bis 4 Kohlenstoffatome enthaltenden Percarbonsäure in Gegenwart von organischen Lösungsmitteln bei einer Temperatur von 0 bis 100°C umsetzt, anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem Lösungsmittel abdestilliert.

2. Verfahren nach Anspruch 1, dadurch gekennzeichnet, daß man die Umsetzung in Gegenwart von 1,2-Dichlorpropan, Tetrachlorkohlenstoff, Benzol, Chlorbenzol, Cyclohexan und/oder Propionsäureäthylester durchführt.

3. Verfahren nach Anspruch 1 bis 2, dadurch gekennzeichnet, daß man die Percarbonsäure in Form einer Lösung in einem organischen Lösungsmittel verwendet, wobei die Lösung weniger als 2 Gew.-% Wasser, weniger als 1 Gew.-% Wasserstoffperoxid und weniger als 50 ppm Mineralsäure enthält.

4. Verfahren nach Anspruch 1 bis 3, dadurch gekennzeichnet, daß man 0,1 bis 5 Mol Percarbonsäure pro Mol Tetrahydrophthalsäurediglycidylester verwendet.

5. Verfahren nach Anspruch 1 bis 4, dadurch gekennzeichnet, daß man dem Reaktionsgemisch vor oder während der destillativen Aufarbeitung Stabilisatoren zusetzt, welche die Bildung von Hochsiedern und Polymerisaten verhindern.

**Claims**

1. Process for the preparation of 7-oxabicyclo(4.1.0)heptane-3,4-dicarboxylic acid diglycidyl ester, characterised in that tetrahydrophthalic acid diglycidyl ester is reacted with a percarboxylic acid containing 3 to 4 carbon atoms in the presence of organic solvents at a temperature of 0 to 100°C, the resulting reaction mixture is then worked up by distillation, the carboxylic acid formed from the percarboxylic acid being separated off by adding an additive to the reaction mixture which has a boiling point between that of the carboxylic acid forming from the percarboxylic acid and that of the constituent of the reaction mixture which has the next highest boiling point and distilling off the carboxylic acid together with the solvent.

2. Process according to Claim 1, characterised in that the reaction is carried out in the presence of 1,2-dichloropropane, carbon tetrachloride, benzene, chlorobenzene, cyclohexane and/or ethyl propionate.

3. Process according to Claims 1 to 2, characterised in that the percarboxylic acid is used in the form of a solution in an organic solvent, the solution containing less than 2% by weight of water, less than

1% by weight of hydrogen peroxide and less than 50 ppm of mineral acid.

4. Process according to Claims 1 to 3, characterised in that 0.1 to 5 mols of percarboxylic acid are used per mol of tetrahydrophthalic acid diglycidyl ester.

5. Process according to Claims 1 to 4, characterised in that stabilisers which prevent the formation of high-boiling constituents and polymers are added to the reaction mixture before or during the working up by distillation.

**Revendications**

1. Procédé de préparation du 7-oxabicyclo(4.1.0)heptane-3,4-dicarboxylate de diglycidyle caractérisé en ce que l'on fait réagir le tétrahydrophthalate de diglycidyle avec un acide percarboxylique contenant de 3 à 4 atomes de carbone en présence de solvants organiques à une température de 0 à 100°C, on traite ensuite le mélange de réaction obtenu par distillation en séparant l'acide carboxylique formé à partir de l'acide percarboxylique par adjonction au mélange de réaction d'un additif qui bout entre l'acide carboxylique formé à partir de l'acide percarboxylique et le constituant du mélange de réaction bouillant immédiatement au-dessus, et en distillant l'acide carboxylique avec le solvant.

2. Procédé selon la revendication 1, caractérisé en ce que l'on effectue la réaction en présence de 1,2-dichloropropane, de tétrachlorure de carbone, de benzène, de chlorobenzène, de cyclohexane et/ou de propionate d'éthyle.

3. Procédé selon les revendications 1 à 2, caractérisé en ce que l'on utilise l'acide percarboxylique à l'état de solution dans un solvant organique, cette solution contenant moins de 2% en poids d'humidité, moins de 1% en poids de peroxyde d'hydrogène et moins de 50 ppm d'acide minéral.

4. Procédé selon les revendications 1 à 3, caractérisé en ce que l'on utilise de 0,1 à 5 moles d'acide percarboxylique par mole de tétrahydrophthalate de diglycidyle.

5. Procédé selon les revendications 1 à 4, caractérisé en ce que l'on ajoute au mélange de réaction, avant ou durant le traitement par distillation, des stabilisants qui inhibent la formation de produits à haut point d'ébullition et de polymères.